# EUROPEAN PATENT SPECIFICATION

(11) **EP 4 045 115 B1**
(45) Date of publication and mention of the grant of the patent: **07.10.2026**
(21) Application number: 19816866.8
(22) Date of filing: 17.10.2019
(51) Int. Cl.: A61M 5/50, A61M 5/315, A61M 5/31

(54) **INJECTION END POINT SIGNALLING ASSEMBLY FOR PRE-FILLED SYRINGES**
SPRITZENENDPUNKTSIGNALISIERUNGSANORDNUNG FÜR VORGEFÜLLTE SPRITZEN
ENSEMBLE DE SIGNALISATION DE POINT D'EXTRÉMITÉ D'INJECTION POUR SERINGUES PRÉ-REMPLIES

(43) Date of publication of application: 24.08.2022
(73) Proprietor: Biocorp Production S.A.S., 63500 lssoire (FR)
(72) Inventor: MARCOZ, Alain, 63160 Montmorin (FR)
(74) Representative: Thurgood, Alexander John
(86) International application number: PCT/IB2019/001115
(87) International publication number: WO 2021/094797

(56) References cited:
- EP-A1- 3 184 135
- WO-A1-2013/164291
- WO-A1-2016/128207
- WO-A1-2018/041798
- WO-A1-2019/121607
- CN-A- 108 917 678

## Description

The present invention relates to pre-filled syringes and associated technology. In particular, the present invention relates to a signalling assembly for pre-filled syringes using a near field communications circuit, commonly abbreviated as NFC.

Pre-filled syringes are known per se to the skilled person and are in common use for the administration of a variety of fixed or unit doses of substances, be they medicaments or other substances. For example, pre-filled syringes are commonly used for the administration of drugs such as vaccines for immunisation campaigns and programmes, or for the treatment of long-term pathologies, such as, for example, diabetes, or other disorders which require management with administration of fixed, pre-measured and stored doses of medicaments, for example, anti-venoms used in the treatment of snake or spider bites, or for emergency injections for the treatment or onset of other potentially life-threatening situations, such as acute pain or trauma, myocardial infarct, anaphylaxis, bacterial or toxic shock and the like. The applications for pre-filled syringes are thus widespread and well known.

Such syringes generally comprise:
an elongated hollow syringe body having a proximal extremity and a distal extremity, with a first opening at the proximal extremity and a collar, or flange, projecting outwardly of the hollow syringe body at said proximal extremity around said first opening;
an injection needle mounted, or mountable, at the distal extremity of the hollow elongated syringe body and closing a second opening of the hollow elongated syringe body at said distal extremity;
a controlled amount of injectable material introduced into the hollow body; and
a plunger configured and dimensioned to be inserted into said hollow elongated syringe body via the proximal extremity and corresponding proximal opening of the hollow syringe body, the plunger having a plunger body comprising a stopper located at a distal extremity of the plunger body, and a plunger head located at a proximal extremity of said plunger body.

One of the general problems with such pre-filled syringes is being able to tell when the syringe has actually been used, in order to avoid attempted re-use, or for tracking purposes, for example in order to know whether and how much of the injectable substance has been administered from the pre-filled syringe. To this end, various tracking systems have been associated with such pre-filled syringes in order to attempt to overcome this general problem.

For example, international patent application published as WO2014089086 relates to a method for using an electronic medicament device such as an auto-injector including a medication such as epinephrine for treating anaphylactic shock. The device includes a sensor, an ID tag, such as a RFID, NFC, or other tag for short range wireless communications, such as Bluetooth communications, a memory, a display, and a speaker, as well as a processor and communication interfaces, the processor interconnecting one or more of the components, and the communication interface including an interface for communication via wifi, a mobile carrier network, or satellite. The processor is configured to communicate with at least one remote system such as a mobile phone via the communication interface in response to the occurrence of an event, such as the administration of the medication and expiration of the medication. The sensor detects activation of the device, and includes a frangible element that completes or breaks an electronic circuit when the device is activated. The sensor provides a signal to the ID tag to perform an action in response to use of the auto-injector device, and alters the memory to indicate that the the device is used, along with a log of the time of use. The ID tag also provides information from the auto-injector device to a wireless reader such as a NFC-enabled mobile device, e.g. a mobile phone. The mobile phone reads medicament information that is either printed on the auto-injector device or stored in the auto-injector device memory using RFID, NFC, or other wireless communication.

Similarly, US patent application published as US2019038840 discloses pre-filled syringe comprising a complex arrangement of two antennae, a first, transmission antenna, configured to transmit a control signal to an external device, control electronics connected to the transmission first antenna configured to provide instructions to the transmission antenna to transmit the control signal, and a second, bypass antenna positioned and configured to prevent the control electronics from providing the instructions to the transmission antenna when the bypass antenna is in an undisturbed position and to permit the control electronics to provide the instructions to the transmission antenna when the bypass antenna is displaced from the undisturbed position. The complex arrangement of the two antennae and control electronics is integrated at a proximal end of the syringe plunger and is covered by a press button. The bypass antenna is configured as a physically destructible electric switch, for which electrical contact is broken when the press-button is depressed by the user of the syringe. Depressing the press button causes the electrical contact to the bypass antenna to be destroyed irreparably, activating the primary antenna circuit and signalling the beginning of use of the syringe.

Both of these solutions are more concerned with the security aspect of whether or not the pre-filled syringe still contains a validly usable drug, on the one hand, or whether or not the injection device has been tampered with, on the other hand. None of these prior art documents relate to, or even credibly address, the problem of knowing whether or not the provided unit dose of drug in the pre-filled syringe has been completely expelled or injected. This situation is known as the injection endpoint.

WO2016128207A1 discloses a medicament delivery device comprising a housing accommodating a medicament container, a drive unit acting on the medicament container upon activation, a user operated activation mechanism, an activation preventing mechanism preventing the activation mechanism from being activated, a usage management module operated by a user. The usage management module is attachable to the medicament delivery device. First keying elements are arranged on the medicament delivery device and on the usage management module which interact with each other when attaching the usage management module, and cause a deactivation of the activation preventing mechanism, thereby enabling operation of the activation mechanism.

EP3184135A1 discloses a communication device for transmitting information from a medicament delivery device, and including a translation detection arrangement, configured to detect a translation of at least one physical part of the medicament delivery device by utilization of a resilience and an electrical conductivity for at least one spring of the medicament delivery device, a change of state detection unit, configured to detect at least one change of state for the medicament delivery device based on the detected translation, an activation unit, configured to activate the at least one change of state detection unit based on the at least one detected translation, and a transmission unit configured to provide a wireless transmission of information related to the at least one change of state to an external receiving device.

WO2018041798A1 discloses a supplementary device for an injection device, comprising a body attachable to a housing of the injection device, the body having a first portion facing towards the housing when attached to the housing, at least one light source attached to the body, and a first optical coupling located in or at the first portion, connected to the at least one light source in a light transmissive way and optically connectable to a second optical coupling arranged in or on the housing.

WO2019121607A1 discloses a supplementary device configured to be releasably attached to an injection device, and comprising a wireless communication unit, and a sensor. The supplementary device is configured to activate the sensor in response to receipt of a wireless communication from an external device, and following activation, to use the sensor to detect the start of an injection, and to communicate the start of the injection to the external device.

CN108917678A discloses a wireless transmission sensor for detecting an object state change, comprising a sending antenna transmitting a control signal to an external device, control electronics connected to the sending antenna, configured to provide transmission control signals to the sending antenna, a bypass antenna positioned and structured to prevent the control electronic device from sending any antenna providing instructions when the bypass antenna is in an uninterrupted position. The control electronic device provides instruction of the transmission control signal to the sending antenna when the bypass antenna is disturbed and is removed from a fixed position. A sensing element is formed such that sensor and communication system are integrated in a sealed communication system. When the sensor detects displacement or a state change of the sensing element, the sealed communication system transmits sensor signals.

WO2013164291A1 discloses a drug delivery device comprising a syringe with a body from which a needle extends and an inner sheath, a piston rod comprising a piston and an actuation head, a support sheath for holding the body, the support sheath and actuation head comprising corresponding indicators.

The invention is defined by the accompanying claims.

An injection endpoint signalling assembly is adapted and configured for mounting on, and use with, a pre-filled syringe as described above, in which information relating to the injection endpoint can only be signalled when the syringe plunger has not only reached the end of its maximal distance of prescribed possible travel within the hollow body of the syringe, thereby ensuring that all of the required unit of injectable substance, for example a drug, initially contained therein before injection has been expelled from the syringe, but also in which signalling of the injection end point can only be enabled when the syringe plunger is also prevented from moving away from the end point position, for example, in a substantially different, for example a reverse or opposite, direction of travel to that usually required to expel an injectable substance, such as a drug, during injection.

The injection endpoint signalling assembly is adapted and configured for mounting on, and use with, a pre-filled syringe, the pre-filled syringe comprising:
an elongated hollow syringe body having a proximal extremity and a distal extremity, with a first opening at the proximal extremity and a collar projecting outwardly of the hollow syringe body at said proximal extremity around said first opening;
an injection needle mounted, or mountable, at the distal extremity of the hollow elongated syringe body and closing a second opening of the hollow elongated syringe body at said distal extremity;
an amount of injectable material introduced into the hollow body;
a plunger configured and dimensioned to be inserted into said hollow elongated syringe body via the proximal extremity and corresponding proximal opening of the hollow syringe body, the plunger having a plunger body comprising a stopper located at a distal extremity of the plunger body, and a plunger head located at a proximal extremity of said plunger body;
characterised in that the injection end point assembly comprises:
   a displaceable electrical contact, comprising a displaceable electrical contact applicator (32) and an electrically conducting surface (36), configured to prevent a signalling of an injection end point before the plunger (8) has reached a limit of a permitted extent of a direction of injection travel, and configured to enable the signalling of the injection end point when the plunger (8) has reached the limit of the permitted extent of the direction of injection travel, wherein the displaceable electrical contact establishes an electrical contact via a translational movement of the electrical contact applicator (32) and electrically conducting surface (36), in a direction different to the direction of injection travel, from a first non-contact position in which no electrical contact is established, to a second contact position establishing an electrical contact; and
   a plunger travel locking means (39) configured to prevent the plunger (8) from moving in a direction of travel different to the direction of injection travel once the limit of the permitted extent of the direction of injection travel has been reached ; and
   wherein the displaceable electrical contact (32, 36) forms an electrical contact at the same time as the plunger travel locking means (39) are engaged.

As used in the present specification, the expression "a limit of a permitted extent of a direction of injection travel" is to be understood as relating to an allowed, predetermined and preconfigured maximum length of travel of the plunger within the hollow elongated syringe body and along a longitudinal axis of the syringe body. Generally, such a maximum limit of permitted extent of a direction of injection travel is defined both by the length of the syringe body, and the stopper located at the distal extremity of the plunger coming into abutting contact with an inner surface of the syringe body at the distal extremity of the syringe body. When this occurs, there is little, or substantially no, injectable substance left within the syringe body. The predetermined, or preconfigured limit of permitted extent of a direction of injection travel of the plunger for injectable substances in syringes is per se well documented and known to the skilled person.

Furthermore, the direction of injection travel is to be understood as referring to the direction in which the plunger travels during injection of the injectable substance. Generally, this direction of travel is substantially or wholly in a distal direction towards a distal extremity of the syringe body in order to expel the injectable substance as is known generally by the person skilled in the art, and which corresponds to the general usage parameters of such pre-filled syringes.

As mentioned above, the injection endpoint assembly is configured to prevent a signalling of an injection end point before the plunger has reached a limit of a permitted extent of a direction of injection travel. This expression is to be understood to mean that the injection endpoint assembly is organised in such a way that the establishment of an electrical connection allowing an electric charge or current to flow within the end point assembly is physically prevented from occurring until such time as the plunger has been moved the maximum limit of injection travel, or, in other words, the injection of injectable substance has to all intents and purposes been completed. Some ways in which this may be achieved are described hereinafter as advantageous or preferred objects of the present invention.

The above can be achieved by providing the end point signalling assembly with a displaceable, or movable, electrical contact configured to enable signalling of the injection point. Such a displaceable electrical contact can take a variety of different forms, for example a simple switch mechanism, a biased, or constrained, electrically conducting metal strip, or the like, or advantageously, a movable electrically conducting surface. The displaceable, or movable, electrical contact is generally arranged to be movable or displaced, from a first position within the injection endpoint signalling in which no electric current may pass through a circuit with which the electrical contact interacts, to a second position within the injection endpoint signally assembly in which an electrical contact is made allowing electrical charge or current to flow through the circuit with which the electrical contact interacts.

The displaceable, or movable, electrical contact establishes an electrical contact via a translational movement of an electrical contact applicator, in a direction different to the direction of injection travel, from a first non-contact position in which no electrical contact is established, to a second contact position establishing an electrical contact. The electrical contact applicator is a means for bringing, or applying, the electrical contact to an electrical gap or an electrically isolated area of an electrical circuit located within the injection endpoint signalling assembly, thereby closing the circuit, and allowing current or charge to flow, for example, when a current is applied to the circuit or the circuit is energized in a way to cause current to flow within the circuit.

The contact applicator is movable or displaceable via a translational movement substantially in parallel to a longitudinal axis of the plunger. Alternatively, the electrical contact applicator can be moved or displaced via a rotational movement about the longitudinal axis of the plunger, and/or by a combination of translational and rotational movement. In all of these variants, the displacement movement of the electrical contact applicator is in a direction different to that of the direction of injection travel, and preferably in a direction substantially opposite to the direction of injection travel. In most cases, this will mean that the electrical contact applicator is moved in a proximal direction, as opposed to the distal direction of injection travel of the plunger.

The electrical contact applicator comprises an electrically conducting surface. Such an electrically conducting surface can usefully comprise a conducting material distributed in, or on such a surface, for example by any of a range of techniques known to the skilled person, such as layering, embedding, deposition whether chemical or physical, etching, engraving, doping, and the like. In a particularly advantageous embodiment, the electrically conducting surface located on the electrical contact applicator comprises carbon or metal particles. This electrically conducting surface will form the electrical contact once the applicator has been moved to the appropriate position, and when not in the contact position, will prevent the establishment of any electrical contact allowing current or charge to flow within an electrical circuit located within the injection endpoint signalling assembly.

The contact applicator is located within the plunger head. Whilst the contact applicator can be positioned and configured to function appropriately in virtually any position, it has been found particularly advantageous to locate the electrical contact applicator within the plunger head, as this allows both for an overall reduction in the size of, and additionally a simplification of, the electrical components involved in the endpoint signalling assembly, particularly in the case where a circuit is also provided in or adjacent the plunger head. Furthermore, the movement, whether translational, rotational, or both, of the electrical contact applicator can be accordingly limited to correspondingly short distances, improving overall precision and accuracy in the device, and lowering the risks of potential failure to establish a sufficiently stable electrical contact.

The injection endpoint signalling assembly comprises plunger travel locking means configured to prevent the plunger from moving in a direction of travel different to the direction of injection travel once the limit of the permitted extent of the direction of injection travel has been reached. The plunger travel locking means are designed and configured in such a way that prevents, or substantially prevents, a user from succeeding in moving the plunger, in a direction different to the direction of injection travel, for example, in a reverse or opposite direction to the direction of injection travel. Any attempt to apply excessive force to move the plunger and thereby potentially affect any signalling made by the injection endpoint assembly would cause a degree of damage to the pre-filled syringe device that it would be rendered incapable of functioning again.

The displaceable electrical contact forms an electrical contact at the same time as the plunger travel locking means are engaged. In other words, the various components of the injection endpoint assembly are so arranged, disposed and configured that the displaceable, or movable, electrical contact is moved into an electrically conducting position within the injection endpoint assembly, for example, within or adjacent the plunger head, at the same time as the plunger travel locking means are activated or engaged to prevent a different direction of travel in the plunger to that of the direction of injection travel.

The plunger travel locking means comprise at least one radially outwardly projecting tine, or a plurality of radially outwardly projecting tines, connected to the plunger body. The projecting tines can either be directly formed on the plunger body, for example, at a substantially proximal region of the plunger body, or alternatively, and equally advantageously, they can be connected indirectly to the plunger body via intermediate connecting means.

The one or plurality of radially outwardly projecting tines is connected to the plunger body via an elastically deformable arm, or a corresponding plurality of elastically deformable arms. In such an embodiment, the elastically deformable arms are advantageously made of the same or a similar material to the plunger body itself, and can extend from a distally located region of the plunger body, in a proximal direction towards the proximal extremity of the plunger body. The arms are elastically deformable, or resilient, in a generally radial direction, meaning that they can either move towards the plunger body, or move away from the plunger body in such a radial direction, depending on the radial forces applied to the arms. This allows the arms to be compressed, for example, under application of a radial force from around an outside of the plunger body, where such radial forces act inwardly towards the plunger body, such as might be applied by the syringe body, generally made of a fairly rigid, non-resilient material such as glass or polycarbonate, or other such crystalline or polycrystalline materials known in the art, for example, when the plunger body is pushed through the syringe body during injection. The resilience or elastic deformation of the arms furthermore allows the arms to move radially outwards away from the plunger body in the event that a user attempts to reverse the direction of travel of the plunger. As the arms connect the radially projecting tines to the plunger body, these tines will move in a radially outwards direction if an attempt is made to withdraw the plunger body from the syringe body in a proximal direction, and the tines will form a lock against either the syringe body and/or an area of the injection endpoint assembly which has been configured to receive such tines.

The injection endpoint signalling assembly further comprises displacement means configured to engage with the contact applicator as the plunger is moved in the direction on injection travel, and cause displacement of the contact applicator in a direction different to said direction of injection travel. The displacement means are the means by which the electrical contact applicator are displaced or moved from a first electrically contactless position to a second electrical contact-established position. Various operational alternatives are envisaged for providing such a configuration, as will be described hereinafter, with a preference for reliable and non-complex solutions.

The displacement means are at least partly located on, or integrated into, the collar of the hollow syringe body. For example, the displacement means can be positioned and located separately and fixedly on the collar of the hollow syringe body, for example by gluing or affixing of the displacement means, or alternatively integrated directly as part of the collar, for example during the moulding process of the syringe body.

Advantageously, the displacement means are located on a syringe backstop removably mounted onto the collar of the hollow syringe body. The notion of a syringe backstop will be explained hereinafter. A syringe backstop enlarges the outwardly projecting collar of the syringe body, generally with ergonomically shaped wings. One of the main objectives of the syringe backstop is to facilitate the handling of the syringe due to an increased area destined to receive a user's fingers. This is particularly of use when administering viscous substances, or when the tissue into which the substance, for example the drug, is being injected, generates a counter-resistance to the injection of the drug. A syringe backstop also facilitates use of the syringe by users with impaired motor capacity, through provision of a larger surface area available for prehension by the user when injecting. Most backstops on sale today are made of a plastics material, and are manually clipped to the outwardly projecting collar, or finger flange, of the syringe body, and as such are usually shaped so as to be compatible with various known collar shapes. Such backstops are generally available under different trade names through companies such as Gerresheimer (Gx^{®} backstops), and Becton Dickinson (BD backstops), to name but two.

As mentioned above, the displacement means can be located on a syringe backstop or directly integrated into, or affixed to, the collar of the syringe body the displacement means comprise a raised arcuate profile. In all variants, the displacement means are generally located so as to comprise a projecting surface that is raised above a proximal surface of the collar, or a proximal surface of the syringe backstop, and which therefore projects from said surface in a proximal direction. Advantageously, the raised, projecting surface is located coaxially around a longitudinal axis of the plunger, on either a proximal surface of the collar, or a proximal surface of the syringe backstop. The raised profile can have a variety of configurations, for example contiguous or non-contiguous areas of raised matter, located in various geometric or non-geometric arrangements on the proximal surface of the collar or the syringe backstop, and made of an elastomeric or resilient material, for example, a silicone elastomer or another polymeric material having an appropriate resilience and/or mechanical resistance to compression. Preferably and advantageously, the raised profile has a substantially arcuate shape. The substantially arcuate shape of material making up the displacement means can further be configured with spurs projecting out at irregular or irregular intervals from the body of arcuate material, giving the projecting surface a substantially serpentine appearance when from the proximal end of the syringe along the longitudinal axis. The projecting spurs facilitate the locating and rotational orientation of the plunger head around the longitudinal axis when the permitted limit of injection travel is reached. The displacement means are thus in a fixed relationship with regard to the syringe body collar or the backstop, and project in a proximal direction. The plunger head is moved in a distal direction during injection travel, so that when the plunger head starts to near the end of its permitted distance of travel, it will come into contact with the projecting raised profile of the displacement means. By continuing to advance the plunger head to its maximum permitted distance of injection travel, the displacement means will further engage with the plunger head, and cause the electrical contact applicator to move in a direction different to the direction of injection travel, preferably following a translational movement along, and in parallel with, the longitudinal axis of the plunger, from a first electrically contactless position to a second position in which electrical contact is established through application of the electrically conducting surface to the isolated area or electrical gap of the circuitry located within the plunger head. Closure of the electric circuit will allow current or charge to flow in the circuit when, say, an energizable communications unit such as a near field communications unit is included in the circuitry.

The plunger travel locking means comprise at least one pair of cooperating and opposite abutment surfaces. The at least one pair of cooperating and opposite facing abutment surfaces each comprise a respective ridged portion of material, for example an annular portion of raised material that can be the same material as that of the element or member to which the ridged portion belongs, or alternatively the ridged portions of material can be added to the element in question, e.g. by gluing, welding, various other standard methods of adhesion, remoulding, and the like.

A first cooperating abutment surface is located on an inner surface of the projecting wall of the syringe backstop, and a second cooperating abutment surface is located on an outer surface of a plunger head. Advantageously, the outer surface of the plunger head is a plunger head cap that substantially covers the plunger head, as will be described in more detail hereinafter.

The injection end point assembly further comprises a wireless communications unit. Such wireless communications units are known per se, and often include one or more known technologies implementing various known communications protocols. Exemplary wireless technologies are those covered by, or implementing standards such as IEEE 802.11 a, b, g, n, ac, ax, also known as "Wi-Fi", cellular radio-frequency communication protocols such as GSM, CDMA, GPRS, 3G, EDGE ,4G, W-CDMA, CDMA2000, HSPDA, LTE, 5G, low powered short distance wireless communications such ZigBee, Bluetooth, TransferJet, IrDA, RFID, Wireless USB, DSRC, Near Field Communication (NFC), and the like.

The wireless communications unit is a near field communications (NFC) circuit. Near field communication (NFC) technology as a derivative or evolution of RFID technology is well known to the skilled person. It is described in detail in the international standards ISO/IEC 14443 and ISO/IEC 18000-3, with the former defining the functioning of ID cards used to store information, such as is found in NFC ID tags and the latter defining RFID communication used by NFC equipped devices. As indicated in the previous sentence, the basis of NFC is to be found in radio-frequency identification, or RFID, technology, which provides for suitably equipped hardware to both supply power to and communicate with an otherwise unpowered, or unenergized, and passive electronic tag using radio waves. Accordingly, the NFC circuit used in the present invention comprises a passive ID tag, which stores a set of information, such as for example, the type of injectable substance, the unit dose, concentration, expiry date, and the like, and any other useful or required information that can be appropriately stored within the limits of such a NFC ID tag. The NFC circuit also comprises suitable and corresponding communication components which would normally enable the NFC circuit, when energized, to exchange said information with another NFC enabled device, such as a smartphone. An antenna forming part of the NFC circuit is also provided, to capture radio waves of the given functional frequency of the NFC protocol and thereby energize the circuit.

Advantageously, the near field communications circuit is located in the plunger head. Advantageously, the near field communications circuit can only be energized when an electrical contact has been established. From the preceding description of the functioning of the NFC circuit and the contact applicator, it will be readily understood that the contact applicator is configured to prevent any energizing charge from being able to flow within the NFC circuit and thereby any signalling, communication or exchange of NFC ID tag information with any other NFC equipped devices for as long as the contact applicator has not established an electrical contact within the communications unit. In this way, the communications unit of the assembly according to the invention remains inactive or disabled until such time as the contact applicator is in the appropriate position and has electrically closed the NFC circuit. For example, where the contact applicator comprises an electrically conducting surface, the NFC circuit will only be able to communicate data when the electrically conducting surface of the contact applicator is in a position to electrically close the circuit, for example, by surface to surface contact of the electrical conducting surface with a gap or isolated area provided in the NFC circuit, thereby enabling electrical charge to flow within the NFC circuit upon energization of the latter. In all other circumstances, the switch remains open, thus no electrical charge can flow within the NFC circuit, and so even if an energization were to occur, for example, by accident, no information from the ID tag can be communicated by the NFC circuit to the NFC equipped device.

The injection endpoint signalling assembly further comprises anti-tamper means configured to prevent tampering of the plunger head when an electrical contact has been established and when the plunger has reached the limit of the permitted extent of the direction of injection travel. In most commercially available pre-filled syringes, the plunger head sticks out beyond the syringe body collar, or the backstop if one is present, even when injection has been completed. This provides an opportunity for a user to try and forcibly reverse the direction of usual travel of the plunger by pulling on the head, or exerting proximal traction in a proximal direction. Accordingly, the assembly is provided with anti-tamper means to prevent such a scenario.

The anti-tamper means comprise a wall located radially outwardly of the displacement means around the longitudinal axis of the plunger, and projecting in a proximal direction. Advantageously, the projecting wall has a proximal extremity that is located adjacent or substantially flush with a proximal surface of the plunger head, when the plunger has travelled its permitted distance or length of injection travel. The substantial alignment of the proximal extremity of the projecting wall with the proximal surface of the plunger head prevents a user, whether by accident or intention, from exerting such a proximally-directed traction, and additionally also prevents any attempted rotation of the plunger head, in a misguided attempt to unscrew it from the rest of the syringe. Even more advantageously, the proximal surface of the plunger head is rounded at a peripheral edge of said proximal surface, providing even fewer possibilities for a user to attempt to dismantle the endpoint signalling assembly.

The plunger head is defined by a substantially circular shaped plunger head plate extending radially outwards from the plunger rod at a proximal end thereof, and having an annular wall extending in a proximal direction from the periphery of said plunger head plate, to form a proximal well with a proximal opening. The height of the annular wall is sufficient to be able to completely contain, position and allow for movement of the electrical contact applicator, for example, from the electrically contactless position to the electrical contact position.

The communications unit including the NFC circuit, can be usefully integrated into a small printed circuit board, for example, of a suitable size and dimension to fit comfortably within or be integrated into, a proximal plunger head cap. The proximal plunger head cap will close the well opening, for example, via a screw-threaded fitting which cooperates and engages with an appropriately configured screw threading provided on an inner surface of the peripheral annular wall forming the well of the plunger head. Integration of the NFC circuit into the proximal cap can be achieved, for example, by suitable moulding around the NFC circuit, which cap is then inserted into the well provided in the plunger head, for example, using the screw threading as described, or alternatively a snap-fit or push-fit coupling of the cap with the well in which the cap has a peripheral annular wall projecting in a proximal direction from the cap and which extends distally beyond the position of the plunger head plate. In other words, in such a configuration, the distally extending annular wall of the plunger head cap has a height greater than the height of the annular wall forming the plunger head well and is intended to abut, via a distal abutting surface of the peripheral distally extending annular wall, the proximal surface of the syringe backstop, once the permitted distance of injection travel has been reached.

Briefly, the injection end point assembly is designed to function as follows:
The cap of the plunger head, located above the bore of the hollow syringe body, houses the communications unit with the NFC circuit. This circuit is initially in an inactivated state due to the provision of an electrical isolation or gap within the circuit. As a result, no communication can be established between the injection endpoint assembly and a registration device, and no data can be passed from the NFC circuit until such time as the electrical isolation or gap is overcome and an electrical contact reestablished allowing an electrical charge to pass through the reestablished circuit. Thus, even if a NFC-equipped device were to approach the plunger head, or vice-versa the syringe was brought near to a corresponding NFC-equipped device such as a smartphone, energization of the NFC circuit would not cause the NFC circuit to become activated. With the circuit thus open until electrical contact is established via the positioning of the contact applicator, no communication of the information stored on the passive ID tag can occur.

When the pre-filled syringe is used, the plunger head gets pressed in a known manner, via the cap, causing the plunger rod to move along the longitudinal axis of the hollow syringe body and inject or eject the injectable substance contained therein. When the plunger reaches the end of the predetermined and permitted distance of travel, the plunger head is locked into an injection end point position by the resilient or elastically deformable arms and radially projecting tines.

At the same time, as the plunger head comes into contact with the displacement means, the latter engage and cause the electrical contact applicator to translate in a proximal direction, with the result that when the plunger is in the locked position, so the contact applicator has been brought into the electrical contact position, and closed the circuit, potentially allowing charge or current to flow. Thus, if the NFC circuit is now energized by another NFC-equipped device, such as by passing a smartphone over the plunger head of the pre-filled syringe, or vice-versa, by passing the plunger head of the pre-filled syringe over a smartphone, then communication of the information stored on the ID tag can occur, thereby signalling, or otherwise indicating, that the end point of the injection has been achieved.

There is further provided a kit of parts adapted and configured for mounting on, and use with, a pre-filled syringe as described herein, wherein the kit of parts comprises an injection end point signalling assembly as described and provided for in the present application. Such a kit of parts allows for adaptation of the end point signalling assembly as described herein to multiple different pre-filled syringes, according to any given manufacturer's specifications.

### BRIEF DESCRIPTION OF THE FIGURES

The invention will now further be described in relation to the figures, provided for illustrative purposes of various embodiments of the invention:
Figure 1 represents a schematic perspective view of a pre-filled syringe with an injection end point signalling assembly according to the invention;
Figure 2 represents a schematic partially cut-away view of a proximal end of a pre-filled syringe and injection end point signalling assembly according to Figure 1;
Figure 3 represents a schematic cross-sectional view of the end point signalling assembly according to Figure 1 mounted on a pre-filled syringe in an initial, ready-to-use position;
Figure 4 represents a schematic cross-sectional view of the injection endpoint signalling assembly according to the invention at the beginning of the injection;
Figure 5 represents a schematic, close-up cross-sectional view of a proximal extremity of the endpoint signalling assembly of Figure 4;
Figure 6 represents a schematic, cross-sectional view of the injection endpoint signalling assembly according to the invention at the end of the injection;
Figure 7 represents a schematic, close-up cross-sectional view of a proximal extremity of the endpoint signalling assembly of Figure 6;
Figures 8 and 9 represent schematic cross-sectional views of an alternative locking means for the injection endpoint signalling assembly according to the invention in a position at the beginning of an injection and at the end of an injection respectively;
Figures 10 and 11 represent the alternative locking means of the injection endpoint signalling assembly of Figures 8 and 9, at the beginning of an injection and the end of an injection respectively.

### EXAMPLE

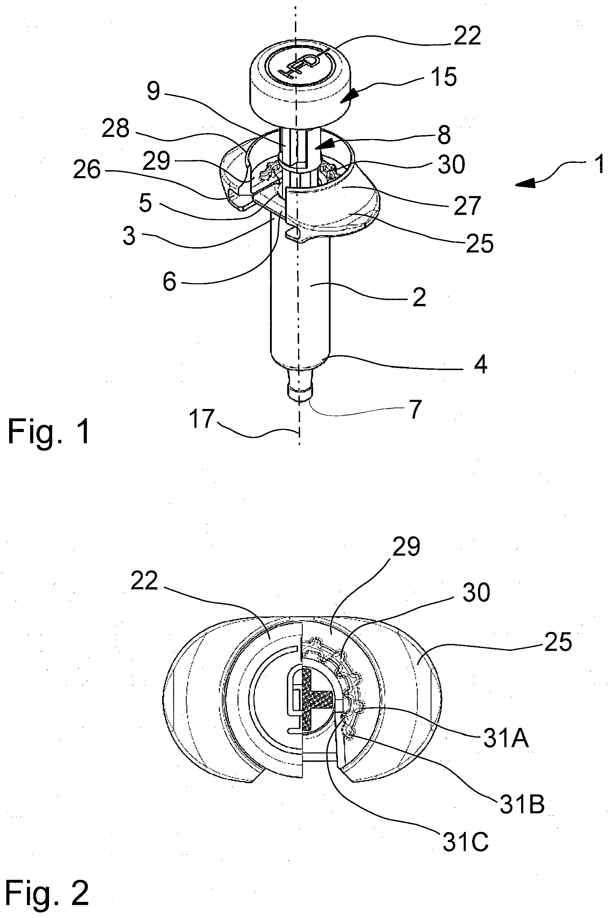

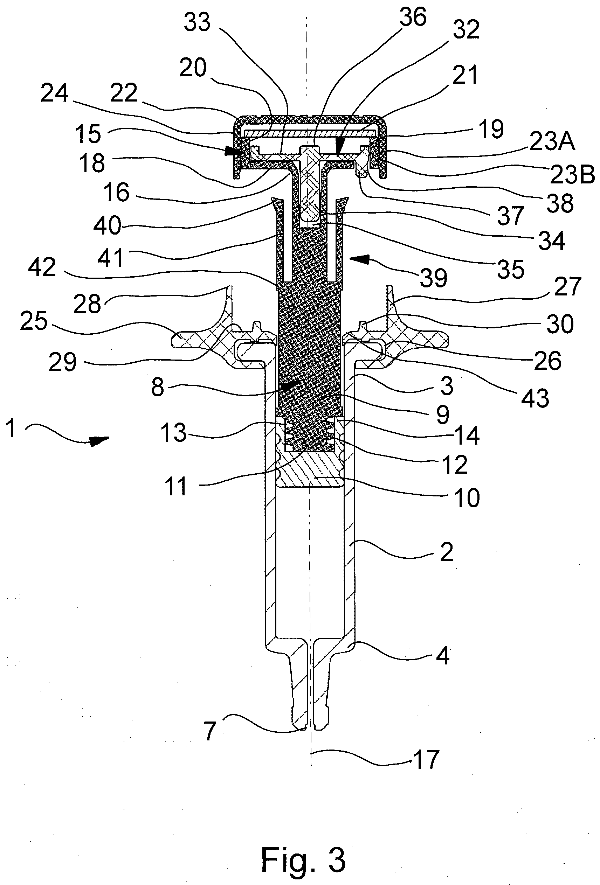

Turning now to the figures, a pre-filled syringe (1) is illustrated in Figures 1 and 3. The pre-filled syringe (1) has an elongated hollow syringe body (2) having a proximal extremity (3) and a distal extremity (4), with a first opening (5) at the proximal extremity (3) and a collar (6), or flange, projecting outwardly of the hollow syringe body (2) at said proximal extremity (3) around said first opening (5). An injection needle (not shown) covered by a needle cap (not shown) is usually mounted at the distal extremity (4) of the hollow elongated syringe body (2) and closes a second, distal opening (7) of the hollow elongated syringe body (2) at said distal extremity (4). A controlled amount of injectable material (not shown) such as a drug in liquid or form, is introduced into the hollow body (2) during assembly of the syringe components.

A plunger (8) is configured and dimensioned to be inserted into the hollow elongated syringe body (2) via the proximal extremity (3) and corresponding proximal opening (5) of the hollow syringe body (2), the plunger (8) having a plunger body or rod (9) comprising a stopper (10) located at a distal extremity (11) of the plunger body (9). The stopper (10) can be connected in a known way to the plunger body (9), for example, through the provision of a screw threaded projection (12) at the distal extremity (11) of the plunger body (9), and a corresponding screw-threaded bore (13) provided inside the stopper (10) at a proximal extremity thereof (14). The plunger body (9) further has a plunger head (15) located at a proximal extremity (16) of said plunger body (9). The plunger (8) and syringe body (2) are in substantial longitudinal alignment along a central longitudinal axis (17) of the syringe body (2).

The plunger head (15) has a substantially circular plate (18) extending radially outwards from the proximal extremity (16) of the plunger body (9). A peripheral annular wall (19) is located on the plate (18) and extends from the plate (18) in a proximal direction forming a well (20). The well is closed at its proximal extremity by a communications unit (21) comprising an NFC circuit, illustrated in the figures as a disc located upon the proximal extremity of the peripheral annular wall (19) of the well (20).

A plunger cap or cover (22) closes the well (20) and covers both the well (20) and the communications circuit (21), the plunger cap (22) being provided with fitting means (23A, 23B) to prevent the cap (22) from falling off or detaching from the well (20), for example a push-fit or snap-fit coupling consisting of an annular groove (23A) provided on an inside surface of a distally projecting annular wall (24) of the cap (22) and a corresponding and mating annular ridge (23B) provided on an outside surface of the peripheral annular wall (19) projecting from the plate (18) of the well (20).

The injection end point assembly further comprises a backstop (25), which is located on the flange or collar (6) of the syringe body (2). Most commercially available backstops (25) comprise a disk-shaped body with a central opening, adapted for receiving the syringe body and configured to enable clip-fit or push-fit of the backstop body onto the collar (6). To this end, the backstops generally comprise a corresponding seating groove (26), and moulded shoulders, or other projections to enable the backstop (25) to be appropriately fitted to a variety of different shaped collars (6), depending on the type of syringe to which the backstop is mounted. In the present example, the backstop (25) further comprises a substantially annular shaped peripheral wall (27) extending in a proximal direction from the backstop body and terminating in a proximal extremity (28), intended to serve as an anti-tamper means, and which will be described in more detail herein.

Also visible in Figures 1, 2, and 3 is a proximal surface (29) of the backstop (25) on which has been affixed or integrated therewith, for example, via deposition or moulding, are displacement means (30) exemplified here as a raised profile (30) which projects in a distal direction. The raised profile (30) is substantially arcuate in shape around the central opening of the backstop body, and is thus also located radially around the longitudinal axis (17). The raised profile of the displacement means (30) in the illustrated example is substantially contiguous and can be provided with spurs (31A, 31B, 31C) which project outwardly from the raised profile, lending an overall serpentine shape to the displacement means.

The displacement means (30) provide a main function of displacement for an electrical contact applicator (32) which is located within the well (20) formed by the plate (18) and peripheral annular wall (19). The electrical contact applicator (32) is a movable, preferably translatable, member, such as a disk or plate (33), in coaxial alignment with the longitudinal axis (17). The disk (33) is mounted on a central rod (34) that is in alignment with the longitudinal axis (17), which rod (34) is slidingly located in a bore (35) that extends from the bottom of the plate (18) in a proximal direction into the body (9) of the plunger (8). The dimensions of the rod (34) and bore (35) are so configured that the rod can not slide of its own free motion within the bore, rather the rod (34) must be constrained into movement by application of force to be able to move slidingly within the bore (35). The central rod (34) also extends in a proximal direction above a proximal surface of the disk (33) to provide at least one proximal electrical contact surface (36), which can comprise an electrically conducting layer, such as a layer of deposited carbon, or an electrically conducting metal, either in elemental form or as a matrix of electrically conducting materials, deposited on, or integrated into the proximal surface. If desired or appropriate, an at least one alternative or further electrical contact surface can optionally be provided at one or more peripheral locations situated radially of the central electrical contact surface (36) on corresponding projections extending proximally from the disk (33).

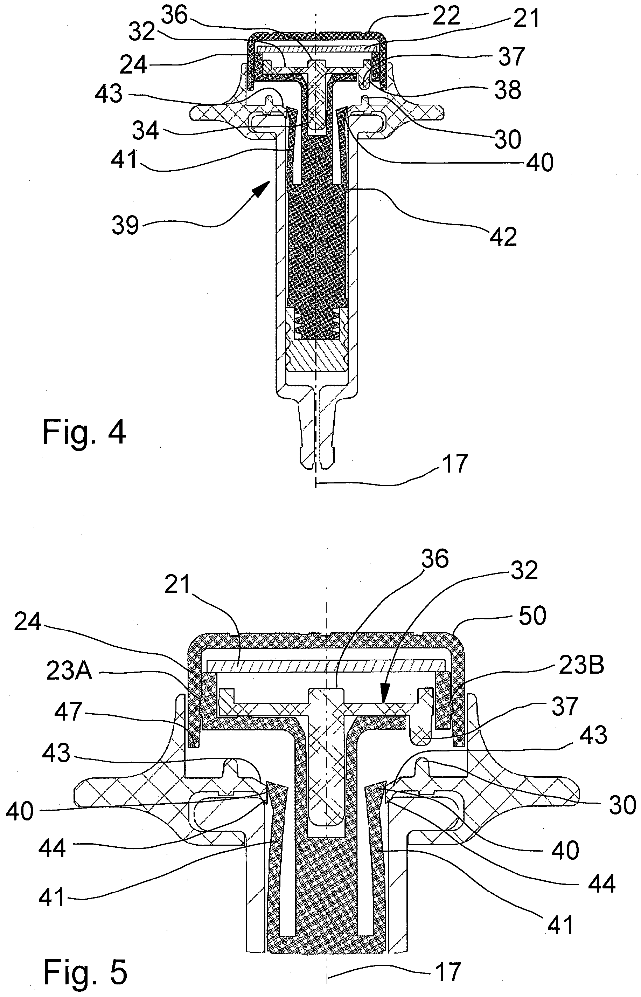

In a first position, when the pre-filled syringe is either in a ready to use state, as in Figure 3, or at the beginning of an injection, as illustrated in Figures 4 and 5, the electrical contact applicator (32) is in a first electrically contact-less position, seated at the bottom of the well, with the rod (34) extending substantially into the bore (35) such that a distal extremity of the rod almost touches a proximal facing surface of the distal extremity of the bore (35). Seating of the applicator (32) can further be provided by a projection (37) which extends distally from the disk (33) in a radially spaced apart relationship to the rod (34) through an opening (38) provided in the plate (18). In this position, the distal facing projection (37) extending from disk (33) is situated in alignment with the raised profile of the displacement means (30), but physically spaced apart from the displacement means.

The communications unit (21), generally comprising a disk-shaped circuit board, a passive NFC circuit including an ID tag included in the circuit board, an antenna, for example, distributed in a spiral configuration around the NFC circuit and located around a peripheral edge of the circuit board, also has an electrical gap or isolation area, provided on a distal face of the circuit board, for example in a central position of the circuit board and in axial alignment with both the longitudinal axis (17) and the electrical contact surface (36), or alternatively and/or additionally, at a peripheral edge of the circuit board to be aligned with the alternative and/or additional electrical contact surfaces provided on further peripheral and/or radially located proximally facing projections extending from the disk (33). In this initial ready-to-use position, as illustrated by Figure 3, or at the beginning of an injection, as illustrated in Figures 4 and 5, the circuit remains in an open state, preventing any flow of electrical charge or current within the circuit, even in the presence of an applied RF energization, such as the approach of a suitably equipped smartphone having and NFC circuit.

Figures 3, 4 and 5 further illustrate the presence of plunger travel locking means (39) provided on the plunger (9). The plunger travel locking means (39) comprise at least one radially outwardly projecting tine (40), or a plurality of radially outwardly projecting tines, connected to the plunger body (9). The projecting tines can either be directly formed on the plunger body, for example, at a substantially proximal region of the plunger body, or alternatively, and equally advantageously, they can be connected indirectly to the plunger body via intermediate connecting means, such as an arm (41) as illustrated in the figures. The arm (41) is elastically deformable. In such an embodiment, the elastically deformable arms are advantageously made of the same or a similar material to the plunger body itself, and can extend from a distally located region, such as a shoulder (42) provided on the plunger body (9), in a proximal direction towards the proximal extremity of the plunger body. The arms are elastically deformable, or resilient, in a generally radial direction, meaning that they can either move towards the plunger body, or move away from the plunger body in such a radial direction, depending on the radial forces applied to the arms. As illustrated in Figures 4 and 5, upon beginning of the injection, the plunger body (9) is pushed into the bore of the syringe body and the relatively more rigid walls of the syringe body cause the arms (41) to be compressed radially inwardly towards the plunger body (9). In addition, the tines (40) come into contact with a proximally facing inwardly sloping surface (43) provided on the disk body of the backstop (25), which proximally facing inwardly sloping surface projects over the collar (6) and at least partially into the bore of the syringe body, forming a locking shoulder (44). This is situation is particularly illustrated by Figure 5.

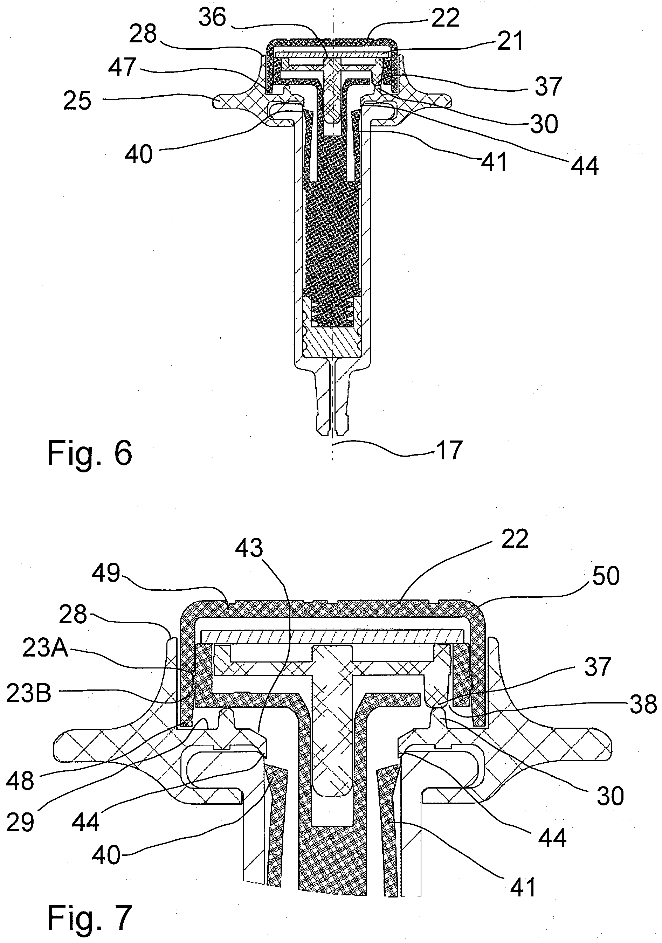

Figures 6 and 7 illustrate the relative positions of the components of the injection endpoint signally assembly at the end of injection. As further injection pressure is applied, the plunger body (6) moves in a distal direction, and the resistance of the inwardly sloping shoulders (43), coupled with the resilience or elastic deformation of the arms (41), causes the tines (40) to be moved via deformation of the arms in a radially inwards direction, thereby forcing the tines over the inwardly sloping surface (43) of the locking shoulder (44) into the bore of the syringe body. The tines (40) therefore come to bear in friction contact on the inside wall of the syringe body. The friction contact between the tines (40) and the inside wall of the syringe body is generally sufficient to prevent withdrawal of the plunger body (9) if a retracting force on the plunger body (9) is exerted in a direction opposite to the injection direction. However, in order to ensure that this can not occur, the locking shoulder (44) formed by the inwardly sloping surface (43) which projects at least partly into the bore of the syringe body actively prevents the plunger from being withdrawn, as the tines (40) abut against the projecting area of the locking shoulder (44) and the natural tendency of the elastically deformable arms (41) to move the tines (40) radially outwardly only serves to increase the locking effect. At the end of injection therefore, the assembly is essentially prevented, or locked from moving in a direction different to that of the direction of injection travel.

At the same time as the plunger body is moved distally during injection, plate (18) of the plunger body (9) is moved towards the raised profile of the displacement means (30). Further progression of the injection causes the projection (37) extending distally through opening (38) to come into abutting contact with the raised profile of the displacement means (30). Continued distal movement of the plunger exerts a sufficient force to overcome any resistance to effort provided for in the dimensioning of the rod (34) and bore (35), such that the projection (37) which is fixedly connected to the disk (33) of the electrical contact applicator (32), causes the contact applicator to be moved, or translated, from the first contactless position, in a direction opposite to that of the direction of injection travel, towards the second position in which an electrical connection will be established in the communications circuit. When the plunger has reached its maximum extent of permitted injection travel, the disk (33) will have been moved in an opposite direction by the interplay of the distal projection (37) and raised profile of the displacement means (30) which is sufficient to bring the electrically conducting contact surface (36) into contact with the electrical isolation area or electrical gap in the circuit, and thereby close that circuit.

In this second position therefore, electrical contact is both established in the circuit, and the endpoint signalling assembly is locked in position preventing any accidental or wilful displacement of both the plunger (8) and the electrical contact applicator (32). Closure of the circuit allows the passive NFC circuit to function when energized by an appropriate external radio frequency such as when a NFC-equipped device, for example, a smartphone or tablet or other NFC reader, is brought in sufficiently close proximity to the plunger head (15), or vice-versa, when the plunger head (15) of the now empty syringe is brought in close proximity to such a NFC-equipped device. Energization of the passive NFC circuit in this way then enables signalling to occur, allowing the data stored in the ID tag of the NFC circuit to be read, and the injection end point to be thus suitably signalled to the NFC-equipped device.

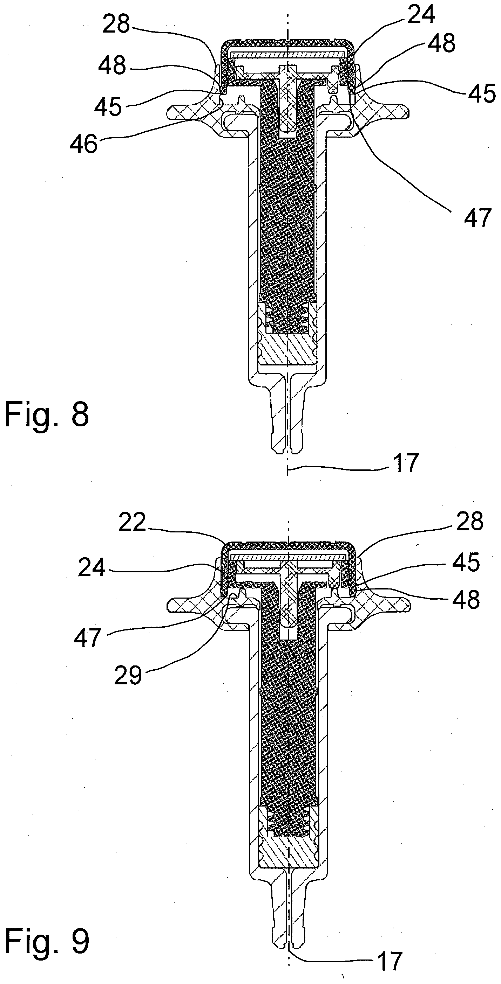

As is also apparent from the various figures, the distally projecting annular wall (24) of plunger head cap (21) extends in a distal direction beyond the level or position of the plunger head plate (18). This surplus distance is used to advantage in an alternative embodiment of the locking means provided in the injection endpoint signalling assembly and illustrated in particular in Figures 8,9, 10 and 11. Like numeric references are provided to designate like objects from Figures 1 to 7.

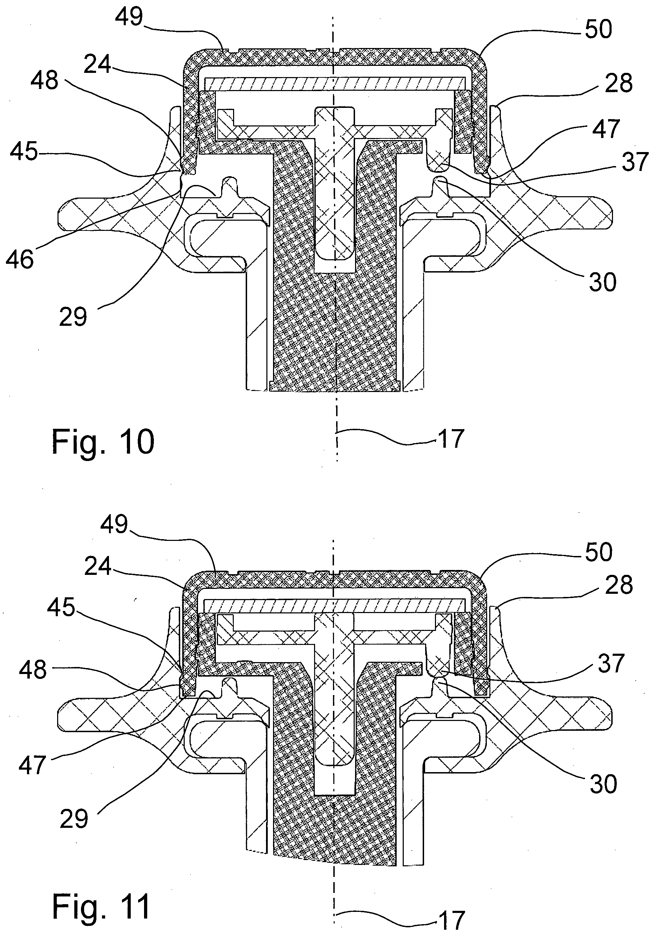

Figures 8 and 10 represent the injection endpoint signalling assembly at the beginning of an injection. Figures 9 and 11 represent the injection endpoint signalling assembly at the end of an injection, when the maximum permitted distance of injection travel has been reached. Turning now to Figures 8 and 10, the most immediately noticeable difference between the embodiments described with regards to Figures 1 to 7 is the absence of radially projecting tines and elastically deformable arms provided on the plunger body. In the embodiment represented by Figures 8 to 11, the plunger travel locking means are provided by at least one pair of opposite-facing abutment surfaces located elsewhere, and in particular a first abutment surface (45) provided on an inner surface (46) of the proximally projecting backstop wall (27). This first abutment surface is usefully provided via a ridged or raised portion of material that is preferably the same as the material from the which the backstop proximally projecting wall is made, but alternatively could be a ridge portion of appropriately elastically deformable or resilient material that is added onto said inner surface, for example, by welding, gluing, remoulding and the like. In Figures 8 and 10, the ridged portion is located at or adjacent to the distal extremity (47) of the distally projecting peripheral annular wall (24) of the cap (22). A second and opposite facing abutment surface (48) is provided on an outer surface of the cap (22) and is usefully provided via a ridged or raised portion of material that is preferably the same as the material from the which the cap peripheral and distally projecting wall is made, but alternatively could be a ridge portion of appropriately elastically deformable or resilient material that is added onto said outer surface, for example, by welding, gluing, remoulding and the like. In Figures 8 and 10 the second abutment surface (48) is located above the first abutment surface (45), but has a distal facing surface of the ridged portion in abutting contact with a proximal facing surface of the ridged portion of the first surface, this configuration preventing the cap from moving in a distal direction without application of an appropriate force to move it in that direction.

Upon injection, a force is applied to the cap (22) in a proximal direction. This force, when sufficiently applied, for example, as a user presses on the cap, enables the distal abutting surface of the ridged portion of the second abutment surface (48) to overcome the resistance opposed by the first abutment surface (45) and move past the ridged portion of the first abutment surface to allow the injection to proceed. Once injection has finished, the plunger and corresponding plunger head, have attained the maximum permitted limit or distance of injection travel. As illustrated by Figures 9 and 11, in this position the second abutment surface (48) is now located proximally of the first abutment surface. Additionally, a proximal surface of the ridged portion of the second abutment surface (48) is now in abutting contact with a distal surface of the ridged portion of the first abutment surface (45). The abutting surfaces prevent the plunger and cap from being moved in a direction opposite to the direction of injection travel, thereby locking the endpoint signalling assembly. The electrical contact applicator is moved in the same way as described for Figures 1 to 7, meaning that at the end of injection, the electrical contact has closed the gap or electric isolation in the communications circuit, allowing signalling to occur through energization of the circuit by the approach of suitably equipped energizing device, such as a smartphone. Additionally, the cap (22), which has a proximal surface (49) adjacent to, or flush with, the proximal extremity (28) of the backstop proximally projecting wall (29), can be provided with rounded corners (50), which together serve as anti-tamper means to prevent attempts to grab the plunger cap to attempt to impose a proximally directed traction force thereon.

## Claims

1. An injection end point signalling assembly adapted and configured for mounting on, and use with, a pre-filled syringe (1), the pre-filled syringe (1) comprising:
an elongated hollow syringe body (2) having a proximal extremity (3) and a distal extremity (4), with a first opening (5) at the proximal extremity (3) and a collar (6) projecting outwardly of the hollow syringe body (2) at said proximal extremity (3) around said first opening (5);
an injection needle mounted, or mountable, at the distal extremity (4) of the hollow elongated syringe body (2) and closing a second opening (7) of the hollow elongated syringe body (2) at said distal extremity (4);
an amount of injectable material introduced into the hollow body (2);
a plunger (8) configured and dimensioned to be inserted into said hollow elongated syringe body (2) via the proximal extremity (3) and corresponding proximal opening (5) of the hollow syringe body (2), the plunger (8) having a plunger body (9) comprising a stopper (10) located at a distal extremity (11) of the plunger body (9), and a plunger head (15) located at a proximal extremity (16) of said plunger body (9);
**characterised in that** the injection end point assembly comprises:
a displaceable electrical contact, comprising a displaceable electrical contact applicator (32) and an electrically conducting surface (36), configured to prevent a signalling of an injection end point before the plunger (8) has reached a limit of a permitted extent of a direction of injection travel, and configured to enable the signalling of the injection end point when the plunger (8) has reached the limit of the permitted extent of the direction of injection travel, wherein the displaceable electrical contact establishes an electrical contact via a translational movement of the electrical contact applicator (32) and electrically conducting surface (36), in a direction different to the direction of injection travel, from a first non-contact position in which no electrical contact is established, to a second contact position establishing an electrical contact; and
a plunger travel locking means (39) configured to prevent the plunger (8) from moving in a direction of travel different to the direction of injection travel once the limit of the permitted extent of the direction of injection travel has been reached ; and
wherein the displaceable electrical contact (32, 36) forms an electrical contact at the same time as the plunger travel locking means (39) are engaged.

2. Injection end point signalling assembly according to claim 1, wherein the plunger travel locking means (39) comprise at least one radially outwardly projecting tine (40), or a plurality of radially outwardly projecting tines, connected to the plunger body.

3. Injection end point signalling assembly according to claim 2, wherein the one or plurality of radially outwardly projecting tines (40) is connected to the plunger body (9) via an elastically deformable arm (41), or a corresponding plurality of elastically deformable arms.

4. Injection end point signalling assembly according to claim 2 or claim 3, wherein the plunger travel locking means (39) comprise at least one pair of cooperating and opposite abutment surfaces (45, 48).

5. Injection end point signalling assembly according to claim 4, wherein the at least one pair of cooperating and opposite facing abutment surfaces (45, 48) each comprise a ridged portion of material.

6. Injection end point signalling assembly according to claim 1, wherein the contact applicator (32) is displaced via a translational movement substantially in parallel to a longitudinal axis of the plunger (8).

7. Injection end point signalling assembly according to claim 1, wherein the contact applicator (32) is located within the plunger head (15).

8. Injection end point assembly according to claim 1, further comprising displacement means (30) configured to engage with the contact applicator (32) as the plunger (8) is moved in the direction on injection travel, and cause displacement of the contact applicator (32) in a direction different to said direction of injection travel.

9. Injection end point signalling assembly according to claim 8, wherein the displacement means (30) are at least partly located on, or integrated into, the collar (6) of the hollow syringe body (2).

10. Injection end point signalling assembly according to claim 8, wherein the displacement means (30) are located on a syringe backstop (25) removably mounted onto the collar (6) of the hollow syringe body (2).

11. Injection end point signalling assembly according to claim 8, wherein the displacement means (30) comprise a raised arcuate profile (30).

12. Injection end point signalling assembly according to claim 11, wherein the raised arcuate profile (30) is located coaxially around a longitudinal axis of the plunger (8), on either a proximal surface of the collar (6), or a proximal surface of the syringe backstop (25).

13. Injection end point signalling assembly according to claim 10, further comprising anti-tamper means (27, 28, 29, 50) configured to prevent tampering of the plunger head (15) when an electrical contact has been established and when the plunger (8) has reached the limit of the permitted extent of the direction of injection travel.

14. Injection end point signalling assembly according to claim 13, wherein the anti-tamper means comprise a wall (27) located radially outwardly of the displacement means (30) around the longitudinal axis of the plunger (8), and projecting in a proximal direction.

15. Injection end point signalling assembly according to claim 13, wherein the projecting wall (27) has a proximal extremity that is located adjacent or substantially flush with a proximal surface (49) of the plunger head (15).

16. Injection end point signalling assembly according to claim 13, wherein a first cooperating abutment surface (45) is located on an inner surface of the projecting wall (27) of the syringe backstop (25), and a second cooperating abutment surface (45) is located on an outer surface of the plunger head (15).

17. Injection end point signalling assembly according to any one of the preceding claims, wherein the injection end point assembly comprises a wireless communications unit (21) which is a near field communications (NFC) circuit located in the plunger head (15).

18. Injection end point signalling assembly according to claim 17, wherein the near field communications circuit (21) is configured to be energized only when an electrical contact has been established.

19. Kit of parts adapted and configured for mounting on, and use with, a pre-filled syringe (1), comprising an injection end point signalling assembly according to any one of claims 1 to 18.

## Patentansprüche

1. Anordnung zur Signalisierung eines Injektionsendpunkts, die für die Montage an und den Einsatz mit einer Fertigspritze (1) ausgelegt und konfiguriert ist, wobei die Fertigspritze (1) Folgendes umfasst:
einen länglichen, hohlen Spritzenkörper (2) mit einem proximalen Ende (3) und einem distalen Ende (4), wobei sich am proximalen Ende (3) eine erste Öffnung (5) befindet und am proximalen Ende (3) um die erste Öffnung (5) herum ein Kragen (6) aus dem hohlen Spritzenkörper (2) nach außen vorsteht;
eine Injektionsnadel, die am distalen Ende (4) des länglichen hohlen Spritzenkörpers (2) angebracht ist oder angebracht werden kann und eine zweite Öffnung (7) des länglichen hohlen Spritzenkörpers (2) an diesem distalen Ende (4) verschließt;
eine Menge an injizierbarem Material, die in den hohlen Körper (2) eingebracht ist;
einen Kolben (8), der so ausgebildet und dimensioniert ist, dass er über das proximale Ende (3) und die entsprechende proximale Öffnung (5) des hohlen, länglichen Spritzenkörpers (2) in diesen eingeführt werden kann, wobei der Kolben (8) einen Kolbenkörper (9) aufweist, der einen an einem distalen Ende (11) des Kolbenkörpers (9) angeordneten Stopfen (10) und einen an einem proximalen Ende (16) des Kolbenkörpers (9) angeordneten Kolbenkopf (15) umfasst;
**dadurch gekennzeichnet, dass** die Anordnung zur Signalisierung eines Injektionsendpunkts umfasst:
einen verschiebbaren elektrischen Kontakt, der einen verschiebbaren elektrischen Kontaktapplikator (32) und eine elektrisch leitende Oberfläche (36) umfasst, der so ausgelegt ist, dass er die Signalisierung eines Injektionsendpunkts verhindert, bevor der Kolben (8) eine Grenze der zulässigen Weite einer Injektionsbewegungsrichtung erreicht hat, und der so ausgelegt ist, dass er die Signalisierung des Injektionsendpunkts ermöglicht, wenn der Kolben (8) die Grenze der zulässigen Weite der Injektionsbewegungsrichtung erreicht hat, wobei der verschiebbare elektrische Kontakt durch eine Translationsbewegung des elektrischen Kontaktapplikators (32) und der elektrisch leitenden Oberfläche (36) in einer von der Injektionsbewegungsrichtung abweichenden Richtung von einer ersten kontaktlosen Position, in der kein elektrischer Kontakt hergestellt ist, in eine zweite Kontaktposition, in der ein elektrischer Kontakt hergestellt wird, einen elektrischen Kontakt herstellt; und
eine Kolbenwegverriegelungseinrichtung (39), die so ausgebildet ist, dass sie verhindert, dass sich der Kolben (8) in eine von der Injektionsbewegungsrichtung abweichende Bewegungsrichtung bewegt, sobald die Grenze der zulässigen Weite der Injektionsbewegungsrichtung erreicht ist; und
wobei der verschiebbare elektrische Kontakt (32, 36) einen elektrischen Kontakt herstellt, sobald die Kolbenwegverriegelungseinrichtung (39) in Eingriff steht.

2. Anordnung zur Signalisierung eines Injektionsendpunkts gemäß Anspruch 1, wobei die Kolbenwegverriegelungseinrichtung (39) mindestens einen radial nach außen ragenden Zinken (40) oder mehrere radial nach außen ragende Zinken umfasst, die mit dem Kolbenkörper verbunden sind.

3. Anordnung zur Signalisierung eines Injektionsendpunkts gemäß Anspruch 2, wobei die eine oder die mehreren radial nach außen ragenden Zinken (40) über einen elastisch verformbaren Arm (41) oder eine entsprechende Anzahl elastisch verformbarer Arme mit dem Kolbenkörper (9) verbunden ist bzw. sind.

4. Anordnung zur Signalisierung eines Injektionsendpunkts gemäß Anspruch 2 oder Anspruch 3, wobei die Kolbenwegverriegelungseinrichtung (39) mindestens ein Paar zusammenwirkender und einander gegenüberliegender Anschlagflächen (45, 48) umfasst.

5. Anordnung zur Signalisierung eines Injektionsendpunkts gemäß Anspruch 4, wobei das mindestens eine Paar zusammenwirkender und einander gegenüberliegender Anschlagflächen (45, 48) jeweils einen geriffelten Materialabschnitt umfasst.

6. Anordnung zur Signalisierung eines Injektionsendpunkts gemäß Anspruch 1, wobei der Kontaktapplikator (32) durch eine Translationsbewegung im Wesentlichen parallel zu einer Längsachse des Kolbens (8) verschoben wird.

7. Anordnung zur Signalisierung eines Injektionsendpunkts gemäß Anspruch 1, wobei sich der Kontaktapplikator (32) innerhalb des Kolbenkopfs (15) befindet.

8. Anordnung zur Signalisierung eines Injektionsendpunkts gemäß Anspruch 1, die ferner eine Verschiebungseinrichtung (30) umfasst, die so ausgebildet ist, dass sie mit dem Kontaktapplikator (32) in Eingriff kommt, wenn der Kolben (8) in Injektionsbewegungsrichtung bewegt wird, und eine Verschiebung des Kontaktapplikators (32) in eine Richtung bewirkt, die von der genannten Injektionsbewegungsrichtung abweicht.

9. Anordnung zur Signalisierung eines Injektionsendpunkts gemäß Anspruch 8, wobei die Verschiebungseinrichtung (30) zumindest teilweise an dem Kragen (6) des hohlen Spritzenkörpers (2) angeordnet oder in diesen integriert ist.

10. Anordnung zur Signalisierung eines Injektionsendpunkts gemäß Anspruch 8, wobei die Verschiebungseinrichtung (30) an einem Spritzenanschlag (25) angeordnet sind, der abnehmbar an dem Kragen (6) des hohlen Spritzenkörpers (2) angebracht ist.

11. Anordnung zur Signalisierung eines Injektionsendpunkts gemäß Anspruch 8, wobei die Verschiebungseinrichtung (30) ein erhabenes, bogenförmiges Profil (30) umfassen.

12. Anordnung zur Signalisierung eines Injektionsendpunkts gemäß Anspruch 11, wobei sich das erhabene, bogenförmige Profil (30) koaxial um eine Längsachse des Kolbens (8) herum entweder an einer proximalen Oberfläche des Kragens (6) oder an einer proximalen Oberfläche des Spritzenanschlags (25) befindet.

13. Anordnung zur Signalisierung eines Injektionsendpunkts gemäß Anspruch 10, die ferner Manipulationsschutzvorrichtungen (27, 28, 29, 50) umfasst, die so ausgelegt sind, dass sie eine Manipulation des Kolbenkopfs (15) verhindern, wenn ein elektrischer Kontakt hergestellt wurde und wenn der Kolben (8) die Grenze der zulässigen Weite der Injektionsbewegungsrichtung erreicht hat.

14. Anordnung zur Signalisierung eines Injektionsendpunkts gemäß Anspruch 13, wobei die Manipulationsschutzvorrichtungen eine Wand (27) umfassen, die radial außerhalb der Verschiebungseinrichtung (30) um die Längsachse des Kolbens (8) angeordnet ist und in proximaler Richtung vorsteht.

15. Anordnung zur Signalisierung eines Injektionsendpunkts gemäß Anspruch 13, wobei die vorstehende Wand (27) ein proximales Ende aufweist, das an eine proximale Oberfläche (49) des Kolbenkopfs (15) angrenzt oder im Wesentlichen bündig mit dieser ist.

16. Anordnung zur Signalisierung eines Injektionsendpunkts gemäß Anspruch 13, wobei sich eine erste zusammenwirkende Anschlagfläche (45) an einer Innenfläche der vorstehenden Wand (27) des Spritzenanschlags (25) befindet und sich eine zweite zusammenwirkende Anschlagfläche (45) an einer Außenfläche des Kolbenkopfs (15) befindet.

17. Anordnung zur Signalisierung eines Injektionsendpunkts gemäß einem der vorhergehenden Ansprüche, wobei die Anordnung zur Signalisierung eines Injektionsendpunkts eine drahtlose Kommunikationseinheit (21) umfasst, bei der es sich um eine im Kolbenkopf (15) angeordnete Nahfeldkommunikationsschaltung (NFC) handelt.

18. Anordnung zur Signalisierung eines Injektionsendpunkts gemäß Anspruch 17, wobei die Nahfeldkommunikationsschaltung (21) so konfiguriert ist, dass sie nur dann mit Strom versorgt wird, wenn ein elektrischer Kontakt hergestellt wurde.

19. Teilesatz, der zur Montage an und zur Verwendung mit einer Fertigspritze (1) angepasst und konfiguriert ist, umfassend eine Anordnung zur Signalisierung des Injektionsendpunkts gemäß einem der Ansprüche 1 bis 18.

## Revendications

1. Ensemble de signalisation de fin d'injection adapté et configuré pour montage sur, et utilisation avec, une seringue préremplie (1), la seringue préremplie (1) comprenant :
un corps de seringue creux allongé (2) présentant une extrémité proximale (3) et une extrémité distale (4), une première ouverture (5) située au niveau de l'extrémité proximale (3) et une collerette (6) se projetant vers l'extérieur du corps de seringue creux (2) au niveau de ladite extrémité proximale (3) autour de ladite première ouverture (5) ;
une aiguille d'injection montée, ou montable, au niveau de l'extrémité distale (4) du corps creux allongé de seringue (2) et venant fermer une deuxième ouverture (7) du corps creux allongé de seringue (2) au niveau de ladite extrémité distale (4) ;
une quantité de matériau injectable introduit dans le corps creux (2) ;
un piston (8) configuré et dimensionné de manière à être introduit dans ledit corps creux allongé de seringue (2) via l'extrémité proximale (3) et l'ouverture proximale correspondante (5) du corps creux de seringue (2), le piston (8) présentant un corps de piston (9) comprenant un bouchon (10) situé au niveau d'une extrémité distale (11) du corps de piston (9), et une tête de piston (15) située à une extrémité proximale (16) dudit corps de piston (9) ;
**caractérisé en ce que** l'ensemble de signalisation de fin d'injection comprend :
un contact électrique déplaçable, comprenant un applicateur de contact électrique déplaçable (32) et une surface électriquement conductrice (36), configuré de manière à empêcher une signalisation de fin d'injection avant que le piston (8) n'ait atteint une limite d'étendue permise de direction de déplacement d'injection, et configuré de manière à permettre la signalisation de la fin d'injection lorsque le piston (8) a atteint la limite de l'étendue permise de direction de déplacement d'injection, selon lequel le contact électrique déplaçable établit un contact électrique via un mouvement de translation de l'applicateur de contact électrique (32) et de la surface électriquement conductrice (36), selon une direction qui est différente de celle du déplacement d'injection, depuis une première position hors contact dans laquelle aucun contact électrique n'est établi, dans une deuxième position de contact établissant un contact électrique ; et
un moyen de verrouillage de déplacement du piston (39) configuré de manière à empêcher le piston (8) de se déplacer selon une direction de déplacement différente de celle de la direction de déplacement d'injection une fois la limite de l'étendue permise de déplacement d'injection a été atteinte ; et
selon lequel le contact électrique déplaçable (32, 36) forme un contact électrique au même moment que le moyen de verrouillage de déplacement du piston (39) est engagé.

2. Ensemble de signalisation de fin d'injection selon la revendication 1, selon lequel le moyen de verrouillage de déplacement du piston (39) comprend au moins une dent se projetant radialement vers l'extérieur (40), ou une pluralité de dents se projetant radialement vers l'extérieur, connecté·e·s au corps de piston.

3. Ensemble de signalisation de fin d'injection selon la revendication 2, selon lequel la dent ou la pluralité de dents se projetant radialement vers l'extérieur (40) est connectée au corps de piston (9) via un bras à déformation élastique (41), ou une pluralité correspondante de bras à déformation élastique.

4. Ensemble de signalisation de fin d'injection selon la revendication 2 ou la revendication 3, selon lequel le moyen de verrouillage de déplacement du piston (39) comprend au moins une paire de surfaces de butée coopérantes et opposées (45, 48).

5. Ensemble de signalisation de fin d'injection selon la revendication 4, selon lequel la au moins une paire de surfaces de butée coopérantes et opposées (45, 48) comprend chacune une partie en matériau à nervures.

6. Ensemble de signalisation de fin d'injection selon la revendication 1, selon lequel l'applicateur de contact (32) se déplace via un mouvement de translation sensiblement en parallèle à un axe longitudinal du piston (8).

7. Ensemble de signalisation de fin d'injection selon la revendication 1, selon lequel l'applicateur de contact (32) se situe dans la tête du piston (15).

8. Ensemble de signalisation de fin d'injection selon la revendication 1, comprenant en outre des moyens de déplacement (30) configurés de manière à s'engager avec l'applicateur de contact (32) pendant que le piston (8) se déplace selon la direction de déplacement d'injection, et provoquant le déplacement de l'applicateur de contact (32) selon une direction différente de celle de la direction de déplacement d'injection.

9. Ensemble de signalisation de fin d'injection selon la revendication 8, selon lequel les moyens de déplacement (30) se situent au moins pour partie sur, ou sont intégrés dans, la collerette (6) du corps creux de seringue (2).

10. Ensemble de signalisation de fin d'injection selon la revendication 8, selon lequel les moyens de déplacement (30) se situent sur une butée arrière de seringue (25) montée de manière amovible sur la collerette (6) du corps creux de seringue (2).

11. Ensemble de signalisation de fin d'injection selon la revendication 8, selon lequel les moyens de déplacement (30) comprennent un profil surélevé en forme d'arc (30).

12. Ensemble de signalisation de fin d'injection selon la revendication 11, selon lequel le profil surélevé en forme d'arc (30) est situé de manière coaxiale autour d'un axe longitudinal du piston (8), soit sur une surface proximale de la collerette (6), soit sur une surface proximale de la butée arrière de seringue (25).

13. Ensemble de signalisation de fin d'injection selon la revendication 10, comprenant en outre des moyens anti-sabotage (27, 28, 29, 50) configurés de manière à empêcher le sabotage de la tête de piston (15) lorsqu'un contact électrique a été établi et lorsque le piston (8) a atteint la limite de l'étendue permise de direction de déplacement d'injection.

14. Ensemble de signalisation de fin d'injection selon la revendication 13, selon lequel les moyens anti-sabotage comprennent une paroi (27) située radialement vers l'extérieur des moyens de déplacement (30) autour de l'axe longitudinal du piston (8), et se projetant selon une direction proximale.

15. Ensemble de signalisation de fin d'injection selon la revendication 13, selon lequel la paroi se projetant (27) présente une extrémité proximale qui est située de manière adjacente ou sensiblement en affleurement à une surface proximale (49) de la tête du piston (15).

16. Ensemble de signalisation de fin d'injection selon la revendication 13, selon lequel une première surface de butée coopérante (45) se situe sur une surface intérieure de la paroi se projetant (27) de la butée arrière de seringue (25), et une deuxième surface de butée coopérante (45) se situe sur une surface extérieure de la tête du piston (15).

17. Ensemble de signalisation de fin d'injection selon l'une quelconque des revendications précédentes, selon lequel l'ensemble de signalisation de fin d'injection comprend une unité de communication sans fil (21) sous forme de circuit de communication à champ proche (NFC) situé dans la tête du piston (15).

18. Ensemble de signalisation de fin d'injection selon la revendication 17, selon lequel le circuit de communication à champ proche (21) est configuré de manière à être alimenté en énergie que lorsqu'un contact électrique a été établi.

19. Ensemble d'éléments adapté et configuré pour un montage sur, et en utilisation avec, une seringue préremplie (1), comprenant un ensemble de signalisation de fin d'injection selon l'une quelconque des revendications 1 à 18.
